# EUROPEAN PATENT SPECIFICATION

(11) **EP 2 289 402 B1**
(45) Date of publication and mention of the grant of the patent: **04.04.2018**
(21) Application number: 10174680.8
(22) Date of filing: 31.08.2010
(51) Int. Cl.: A61B 5/0205, A61B 5/113, A61B 5/00, A63B 24/00, G06F 19/00, A61B 5/024, A61B 5/026, A61B 5/0476, A61B 5/0488, A61B 5/08, A61B 5/0402, A61B 5/145, A61B 5/22, A61B 5/0295

(54) **Method and system for interpretation and analysis of physiological, performance, and contextual information**
Verfahren und System zur Interpretation und Analyse von Physiologie-, Leistungs- und Kontextinformationen
Procédé et système pour l'interprétation et l'analyse des informations physiologiques, de performance et contextuelles

(30) Priority: 01.09.2009 US 275634 P; 26.08.2010 US 869625
(43) Date of publication of application: 02.03.2011
(73) Proprietor: Adidas AG, 91072 Herzogenaurach (DE)
(72) Inventor: Derchak, Alexander P., Oxnard, CA 93035 (US)
(74) Representative: Wegner, Hans

(56) References cited:
- WO-A1-01/28420
- WO-A2-2006/009830
- US-A1- 2004 122 334

## Description

### Field of the Invention

The present invention is defined by claims 1 and 6 and relates generally to methods and systems for monitoring physiological and performance characteristics of a subject. More particularly, the invention relates to multivariable analysis of physiological, performance, and contextual information.

### Background of the Invention

Historically, the understanding of disease and the monitoring of patients has been primarily achieved through the evaluation of single variables. The capability to evaluate multiple variables from a subject simultaneously and continuously has the potential to dramatically increase understanding of human physiology through research and to improve the precision of diagnosis and treatment prescription in disease. Moreover, the capability to evaluate multiple variables from an athlete engaged in an athletic activity simultaneously and continuously has the potential to increase understanding of, for example, human physiology, athletic performance progression, and fitness training effectiveness.

Monitoring physiological and performance parameters of a subject can be important in planning and evaluating athletic training and activity. A subject may exercise or otherwise engage in athletic activity for a variety of reasons, including, for example, to maintain or achieve a level of fitness, to prepare for or engage in competition, and for enjoyment. The subject may have a training program tailored to his or her fitness level and designed to help him or her progress toward a fitness or exercise goal. Physiological and performance parameters of a subject can provide useful information about the subject's progression in a training program, or about the athletic performance of the subject. In order to accurately appraise the subject's fitness level or progress toward a goal, it may be useful to determine, monitor, and record various physiological or performance parameters, and related contextual information.

Various methods and systems utilizing heart rate have been introduced to approximate effort and physiological stress during exercise. Convenient, practicable, and comfortable means of measuring pulmonary ventilation in non-laboratory conditions, however, have been scarce. While of good value, heart rate can only give an approximation as to the true physiological state of an athlete or medical patient, as it can be confounded by external factors including, for example, sleep levels, caffeine, depressants, beta blockers, stress levels, hydration status, temperature, etc. Furthermore, accurate use of heart rate to gauge physiological performance requires knowledge of the amount of blood flowing to the muscles, which in turn requires knowledge of the instantaneous stroke volume of the heart as well as the rate of pumping. These parameters can be difficult to determine while a subject is engaging in a physical activity.

In addition, chronic diseases are often expressed by episodic worsening of clinical symptoms. Preventive treatment of chronic diseases may reduce the overall dosage of required medication and associated side effects, and may lower mortality and morbidity. Generally, preventive treatment should be initiated or intensified as soon as the earliest clinical symptoms are detected, in order to prevent progression and worsening of the clinical episode and to stop and reverse the pathophysiological process.

Many chronic diseases cause systemic changes in vital signs, such as, for example, breathing and heartbeat patterns, through a variety of physiological mechanisms. For example, common respiratory disorders, such as asthma, chronic obstructive pulmonary disease (COPD), and cystic fibrosis (CF), are direct modifiers of breathing and/or heartbeat patterns. Other chronic diseases, such as diabetes, epilepsy, and certain heart conditions (e.g., congestive heart failure (CHF)), are also known to modify cardiac and breathing activity. In the case of certain heart conditions, such modifications typically occur because of patho-physiologies related to fluid retention and general cardiovascular insufficiency. Other signs, such as coughing and sleep restlessness, are also known to be of importance in some clinical situations.

Many chronic diseases are associated with characteristic, if sometimes subtle, changes in vital signs. For example, some chronic diseases interfere with normal breathing and cardiac processes during wakefulness and sleep, causing abnormal breathing and heartbeat patterns.

Breathing and heartbeat patterns may be modified via various direct and indirect physiological mechanisms, resulting in abnormal patterns related to the cause of modification. Some respiratory diseases (e.g., asthma) and some heart conditions (e.g., CHF) are direct breathing modifiers. Other metabolic abnormalities (e.g., hypoglycemia and other neurological pathologies affecting autonomic nervous system activity) are indirect breathing modifiers.

Similarly, athletic activity can modify physiological parameters, such as, for example, breathing rate, tidal volume, breathing pattern, heart rate, heart pattern, body temperature, and skin temperature.

Asthma is a chronic disease with no known cure. Substantial alleviation of asthma symptoms is, however, possible via preventive therapy, such as the use of bronchodilators and anti-inflammatory agents. Asthma management presents a serious challenge to the patient and physician as preventive therapies may require constant monitoring of lung function and corresponding adaptation of medication type and dosage.

Asthma episodes usually develop over a period of several days, although they may sometimes seem to appear unexpectedly. The gradual onset of the asthmatic episode provides an opportunity to start countermeasures to stop and reverse the inflammatory process. Early treatment at the pre-episode stage may reduce the clinical episode manifestation considerably, and may even prevent the transition from the pre-clinical stage to a clinical episode altogether.

Two techniques are generally used for asthma monitoring. The first technique, spirometry, evaluates lung function using a spirometer (i.e., an instrument that measures the volume of air inhaled and exhaled by the lungs). Airflow dynamics are measured during a forceful, coordinated inhalation and exhalation effort by the patient into a mouthpiece connected via a tube to the spirometer. A peak-flow meter is a simpler device that is similar to the spirometer, and is used in a similar manner.

The second technique evaluates lung function by measuring nitric-oxide concentration using a dedicated nitric-oxide monitor. The patient breathes into a mouthpiece connected via a tube to the monitor.

Efficient asthma management may require daily monitoring of respiratory function, which is generally impractical, particularly in non-clinical or home environments. Peak-flow meters and nitric-oxide monitors provide a general indication of the status of lung function, however, these monitoring devices do not possess predictive value, and are used as during-episode markers. In addition, peak-flow meters and nitric-oxide monitors require active participation of the patient, which is difficult to obtain from many children and substantially impossible to obtain from infants.

CHF is a condition in which the heart is weakened and unable to circulate blood to meet the body's needs. The subsequent buildup of fluids in the legs, kidneys, and lungs characterizes the condition as congestive. The weakening may be associated with either the left, right, or both sides of the heart, with different etiologies and treatments associated with each type. In most cases, it is the left side of the heart that fails, so that it is unable to efficiently pump blood to the systemic circulation. The ensuing fluid congestion of the lungs results in changes in respiration, including alterations in rate and/or pattern, accompanied by increased difficulty in breathing and tachypnea.

Quantification of such abnormal breathing provides a basis for assessing CHF progression. For example, Cheyne-Stokes Respiration (CSR) is a breathing pattern characterized by rhythmic oscillation of tidal volume with regularly recurring periods of alternating apnea and hyperpnea. While CSR may be observed in a number of different pathologies (e.g., encephalitis, cerebral circulatory disturbances, and lesions of the bulbar center of respiration), it has also been recognized as an independent risk factor for worsening heart failure and reduced survival in patients with CHF.

In CHF, CSR is associated with frequent awakening that fragments sleep, and with concomitant sympathetic activation, both of which may worsen CHF. Other abnormal breathing patterns may involve periodic breathing, prolonged expiration or inspiration, or gradual changes in respiration rate usually leading to tachypnea.

Pulsus paradoxus is a physical sign present in a variety of cardiac and extra-cardiac conditions, and is of valuable diagnostic and prognostic significance. Pulsus paradoxus is generally defined as a fall in systolic blood pressure of over 10 mmHg during inspiration. Pulsus paradoxus has been associated with the following conditions: cardiac tamponade, pericardial effusion, constrictive peri-carditis, restrictive cardiomyopathy, pulmonary embolism, acute myocardial infarction, cardiogenic shock, bronchial asthma, tension pneumothorax, anaphylactic shock, volvulus of the stomach, diaphragmatic hernia, and superior vena cava obstruction.

In bronchial asthma, pulsus paradoxus is of significance because it has often been associated with mild obstructions and can therefore serve as an early warning sign.

Various systems and methods have thus been developed to obtain and interpret physiological and contextual information associated with a subject.

U.S. Patent No. 6,468,234, issued October 22, 2002, describes an apparatus for measuring sleep quality that utilizes sensors incorporated in a sheet that is laid on top of a conventional mattress on which the subject sleeps. The sensors collect information, such as the subject's position, temperature, sound/vibration/movement, and optionally other physical properties.

The apparatus includes one or more layers of arrays of integrated sensors, which can be incorporated in layer pads and then placed on a conventional mattress, one or more controllers coupled with the arrays of integrated sensors in each layer pad for the purpose of acquiring data from the sensors, real-time analysis software for analyzing data acquired by the controller from the array of integrated sensors, interface software for collecting user lifestyle data, lifestyle correlation software for correlating the lifestyle data with the data acquired by the array of sensors, and one or more active components to improve sleep quality based on the data acquired through the sensors and the lifestyle data. The array of sensors provides one or more of the following data: position, temperature, sound, vibration, and movement data.

U.S. Patent No. 6,840,907, issued January 11, 2005, describes a respiratory analysis system for monitoring a respiratory variable of a patient. The system includes a sensor array for accommodating a patient in contact therewith and a processing means. The array has a plurality of independent like sensors for measuring respiratory movement at different locations on the patient to generate a set of independent respiratory movement signals. The processing means receives and processes the movement signals to derive a classification of individual breaths using, for each breath, the respective phase and/or amplitude of each movement sensor signal within the set for that breath.

U.S. Patent No. 6,517,497, issued February 11, 2003, describes techniques for monitoring and/or quantitatively measuring a patient's respiration using a flexible piezoelectric film sensor. The apparatus includes a piezoelectric film that converts acoustical waves generated by the patient's respiration into electrical signals. The piezoelectric film sensor can be used to monitor the respiration of a patient by correlating the sound generated in the patient's airway with respiratory activity. The data generated by the sensor may be further analyzed by a patient monitor to diagnose respiratory conditions.

U.S. Patent No. 5,002,060, issued March 26, 1991, describes a monitoring system adapted to simultaneously monitor cardiac and respiratory rates and characteristics and substantial changes in temperature of a subject. The system uses sensors which are passive and non-invasive, and located remotely from (i.e., completely off of) the subject. Sensor signals are processed in order to provide an alarm accompanied with displayed indication of any irregularities in the cardiac and respiratory rates and characteristics, and substantial changes in temperature.

U.S. Patent No. 6,450,957, issued September 17, 2002, describes a respiration monitoring system that monitors the state of disorder of the respiratory system of a sleeping patient based on the detection of respiratory body movement, without the need to put sensors directly on the patient's body. The system includes weight sensors that produce weight signals attributable to the patient's respiratory body movement. From weight signals having a frequency band of respiration, a respiratory body movement signal is produced. The fall of blood oxygen saturation, which occurs during obstructive apnea of the sleeping patient, is determined based on the variation pattern of the amplitude of the respiratory body movement signal.

U.S. Patent No. 6,790,183, issued September 14, 2004, describes a lung sound diagnostic system for use in collecting, organizing, and analyzing lung sounds associated with the inspiration(s) and expiration(s) of a patient. The system includes a plurality of transducers that may be placed at various sites around the patient's chest. The microphones are coupled to signal processing circuitry and A/D converters that digitize the data and preferably provide the digital data to a computer station. The system may also include application programs for detecting and classifying abnormal sounds. Additionally, the system may include an analysis program for comparing selected criteria corresponding to the detected abnormal sounds with predefined thresholds in order to provide a likely diagnosis. Also described are a system and method for differentiating between the crackles produced by a patient with interstitial pulmonary fibrosis (IPF) from the crackles produced by a CHF patient.

U.S. Patent No. 5,738,102, issued April 14, 1998, describes a system for monitoring and computer analyzing select physiological variables of a patient in real time in order to alert medical personnel to the need for medical treatment or to automatically administer such treatment under computer control. The physiological variables monitored by the system may include lung sounds, respiratory rate and rhythm, heart rate and rhythm, heart sounds, and body temperature. Coded signals relating to the physiological variables are produced and compared with reference versions of same by a decision computer in order to evaluate the patient's condition. If the evaluation indicates medical treatment is needed, the decision computer activates a local and/or a remote alarm to alert medical personnel and/or activates one or more actuators for administering a medical treatment such as the injection or infusion of a drug.

Examples of body sounds that may be detected are respiratory sounds and heart sounds. In the case of the former, the computer produces coded signals representing the rate and rhythm of breathing derived from the respiratory sounds.

The system is described as being able to detect abnormal breathing patterns such as apnea, tachypnea, hyperpnea (e.g., Kussmaul breathing associated with metabolic acidosis), bradypnea, Cheyne-Stokes breathing, ataxic breathing, and obstructive breathing. Coded signals may also be generated from the respiratory sounds that indicate the presence of added lung sounds such as rales associated with pneumonia and pulmonary edema, wheezes associated with obstructive lung disease, and pleural rubs due to inflammation of the pleural membranes.

U.S. Patent No. 6,599,251, issued July 29, 2003, describes non-invasive techniques for monitoring the blood pressure of a subject. A pulse signal is detected at both a first and second location on the subject's body. The elapsed time between the arrival of corresponding points of the pulse signal at the first and second locations is determined. Blood pressure is related to the elapsed time by mathematical relationships.

U.S. Patent Application Publication No. 2004/0133079, published July 8, 2004, describes techniques for predicting patient health and patient relative well-being within a patient management system. One embodiment utilizes an implantable medical device including an analysis component and a sensing component further including a three-dimensional accelerometer, a transthoracic impedance sensor, a cardio-activity sensor, an oxygen saturation sensor, and a blood glucose sensor. One analysis described is detecting changes in transthoracic impedance variation patterns that are indicative of the early occurrence of a new disease state (such as Chronic Obstructive Pulmonary Disease), the onset of an illness (such as asthma), or the progression of a disease (such as DC impedance indicating lung fluid accumulation that corresponds to the progression of heart failure).

U.S. Patent No. 6,454,719, issued September 24, 2002, describes techniques for determining the cardiac condition of a patient by a cardiac monitor apparatus using a respiration parameter such as a current respiration signal or a respiration rate. The variability of the respiration parameter is used to generate a signal indicative of the current heart failure status of the patient, and, more particularly, whether the patient's condition has improved, worsened, or remained unchanged over a predetermined time period. The circuitry for detecting the respiration parameter may be implanted in the patient, for example as part of a pacemaker, while at least some of the analyzing circuitry may be external and remote from the patient. Alternatively the whole device may be implantable.

U.S. Patent No. 6,600,949, issued July 29, 2003, describes a method for monitoring the condition of a heart failure patient using respiration patterns. An implantable or other ambulatory monitor senses the patient's respiratory patterns to identify the presence of periodic breathing or Cheyne-Stokes respiration. In a first embodiment, mechanical changes of the thorax due to breathing are detected and this data is used to recognize hyperventilation and apnea or hypoventilation. In a second embodiment, Cheyne-Stokes respiration is recognized by detecting changes in blood or tissue pH or CO₂ concentration and partial pressure. In another embodiment, changes in pulse amplitude associated with Cheyne-Stokes respiration are detected. Alternating loss and return of respiration-induced amplitude modulation or pulse-interval variation may also be used to identify the presence of Cheyne-Stokes respiration.

In yet another embodiment, modulation of the average heart rate over time is monitored and its absence is used as an indicator of Cheyne-Stokes respiration. This information may be used to warn the patient or healthcare provider of changes in the patient's condition warranting attention.

U.S. Patent No. 6,527,729, issued March 4, 2003, describes a method for monitoring the disease progression of a heart failure patient. An implantable or other ambulatory monitor senses acoustic signals, including heart and lung sounds within the patient. Significant changes in the energy content of either the heart or lung sounds is indicative of a heart failure exacerbation.

U.S. Patent No. 6,015,388, issued January 18, 2000, to Sackner et al. (VivoMetrics, Inc.) describes a method for measuring respiratory drive, including determining a peak inspiratory flow and a peak inspiratory acceleration from a breath waveform derived from rib cage motion and abdominal motion using a plethysmograph or other external respiratory measuring device. The respiratory drive is ascertainable even during complete blockage of the respiratory system. In one embodiment, the peak inspiratory drive is used to initiate inspiration in a mechanical ventilator and for determining an index describing a shape of the waveform for controlling a continuous positive air pressure (CPAP) device.

U.S. Patent No. 6,047,203, issued April 4, 2000, to Sackner et al. (VivoMetrics, Inc.) describes physiological monitoring apparel worn by a monitored individual, the apparel having attached sensors for monitoring parameters reflecting pulmonary function, cardiac function, or the function of other organ systems. In one embodiment, an alarm is generated based on a trend progressing over one to a few hours.

There are several drawbacks associated with the noted prior art systems and methods. A major drawback is that virtually all of the prior art systems (and associated methods) are not adapted to enable continuous, simultaneous, and synchronous capture of signals reflecting multiple physiological, performance, and contextual characteristics from a range of real-world and/or laboratory settings.

### BRIEF SUMMARY OF THE INVENTION

The present invention is directed to methods of data interpretation and inference making that are possible when a multitude of physiological signals are captured continuously (or pseudo continuously), synchronously, and simultaneously. In some embodiments, physiological or performance context is added to any individual data stream when combined with others. For example, an elevated heart rate can be a normal or an abnormal condition. The addition of synchronous respiratory and activity data enables interpretation of the elevated heart rate observation in a way that would be impossible absent the other signals. An elevated heart rate occurring with an increase in respiratory activity and physical activity is a completely normal condition. However, an increase in heart rate and respiratory rate without a concurrent increase in activity indicates physical or emotional stress, and an increase in heart rate without an increase in respiratory rate or activity could signal an impending cardiac arrhythmia or other undesirable sequelae. This sort of interpretive power is only possible when there are multiple signals collected from an individual in a synchronous concurrent manner.

Applications for this interpretive approach enhance both real-time monitoring and the amount and value of the information in research data from a large number of conditions including acute events (e.g., cardiac arrest, respiratory distress, asthma attack, and allergic reactions), psychological events (e.g., panic/anxiety attacks), and chronic disease monitoring (e.g., chronic obstructive pulmonary disease, congestive heart failure, asthma, coronary artery disease, hypertension, diabetes, obesity, depression, and anxiety).

### BRIEF DESCRIPTION OF THE FIGURES

Further features and advantages will become apparent from the following and more particular description of the present invention, as illustrated in the accompanying drawings, in which like referenced characters generally refer to the same parts or elements throughout the views.
FIG. 1 is a schematic illustration of a physiology monitoring system, according to one embodiment of the invention.
FIG. 2 is a schematic illustration of a dual-paired electromagnetic coil arrangement, according to one embodiment of the invention.
FIG. 3 is a side view of a subject, showing the position of the dual-paired electromagnetic coil arrangement of Fig. 2 on the subject, according to one embodiment of the invention.
FIG. 4 is a perspective view of the subject, showing the position of electromagnetic coils on the front of the subject, according to one embodiment of the invention.
FIG. 5 is a plane view of the subject's back, showing the position of electromagnetic coils thereon, according to one embodiment of the invention.
FIGS. 6 and 7 are schematic illustrations of a multiple-paired electromagnetic coil arrangement, according to one embodiment of the invention.
FIG. 8 is a perspective view of a subject, showing the position of the multiple-paired electromagnetic coils shown in FIG. 6 on the front of the subject, according to one embodiment of the invention.
FIG. 9 is a plane view of the subject's back, showing the position of electromagnetic coils thereon, according to one embodiment of the invention.
FIG. 10-12 are schematic illustrations of coil transmission axes provided by several multiple-paired coil embodiments of the invention.
FIG. 13 is a perspective view of a subject, showing alternative positions of the multiple-paired electromagnetic coils shown in FIG. 6 on the front of the subject, according to another embodiment of the invention.
FIG. 14 is a plane view of the subject's back, showing the positioning of three pairs of electromagnetic coils thereon, according to another embodiment of the invention.
FIG. 15 is a plane view of the subject's back, showing alternative positions of the paired electromagnetic coils shown in FIG. 14 thereon, according to another embodiment of the invention.
FIG. 16 is a perspective view of a subject, showing the position of six pairs of electromagnetic coils on the front and one side of the subject, according to another embodiment of the invention.
FIG. 17 is a plane view of the subject's back, showing the position of five pairs of electromagnetic coils on the back and both sides of the subject, according to another embodiment of the invention.

### DETAILED DESCRIPTION OF THE INVENTION

Before describing the present invention in detail, it is to be understood that this invention is not limited to particularly exemplified methods, apparatuses, systems or circuits, as such may, of course, vary. Thus, although a number of methods and systems similar or equivalent to those described herein can be used in the practice of the present invention, exemplary embodiments are described herein.

It is also to be understood that the terminology used herein is for the purpose of describing particular embodiments of the invention only and is not intended to be limiting.

Unless defined otherwise, all technical and scientific terms used herein have the same meaning as commonly understood by one having ordinary skill in the art to which the invention pertains.

As used in this specification and the appended claims, the singular forms "a", "an", and "the" include plural referents unless the content clearly dictates otherwise.

The publications discussed herein are provided solely for their disclosure prior to the filing date of the present application. Nothing herein is to be construed as an admission that the present invention is not entitled to antedate such publication(s) by virtue of prior invention. Further, the dates of publication may be different from the actual publication dates, which may need to be independently confirmed.

### Definitions

The terms "respiratory parameter" and "respiratory characteristic", as used herein, mean and include a characteristic associated with the respiratory system and functioning thereof, including, without limitation, breathing frequency (fB), tidal volume (V_{T}), inspiration volume (V_{I}), expiration volume (V_{E}), minute ventilation (VE), inspiratory breathing time, expiratory breathing time, and flow rates (e.g., rates of change in the chest wall volume). The terms "respiratory parameter" and "respiratory characteristic" further mean and include inferences regarding ventilatory mechanics from synchronous or asynchronous movements of the chest wall compartments.

According to the present invention, flow rates and respiratory accelerations can be determined from a volume signal. Further, numerous inferences regarding ventilatory mechanics can be drawn from the degree of asynchrony in movement occurring among the discrete compartments that make up the chest wall.

The terms "respiratory system disorder", "respiratory disorder", and "adverse respiratory event", as used herein, mean and include any dysfunction of the respiratory system that impedes the normal respiration or ventilation process.

The terms "physiological parameter" and "physiological characteristic", as used herein, mean and include, without limitation, electrical activity of the heart, electrical activity of other muscles, electrical activity of the brain, pulse rate, blood pressure, blood oxygen saturation level, skin temperature, and core temperature.

The terms "spatial parameter" and "spatial characteristic", as used herein, mean and include a subject's orientation and/or movement.

The terms "patient" and "subject", as used herein, mean and include humans and animals.

Pulmonary ventilation, tidal volume, respiratory rate, and other associated respiratory characteristics can provide a reliable and practical measure of oxygen and carbon dioxide transpiration in a living body. Respiratory characteristics are directly connected to exercise effort, physiological stress, and other physiological characteristics. One way to externally determine tidal volume is to measure the change in thoracic volume. Change in thoracic volume is caused by the expansion and contraction of the lungs. As the gas pressure in the lungs at the maxima and minima of the pressure ranges is equilibrated to surrounding air pressure, there is a very close and monotonic relationship between the volume of the lungs and the volume of air inspired.

Accurate measurement of the change in thoracic volume involves measuring the change in the diameter of the chest at the ribcage. Measurement of the change in the diameter of the chest below the ribcage can provide additional accuracy to the measurement. Monitoring changes in the diameter of the chest below the ribcage can account for diaphragm delivered breathing where the contraction and relaxation of the diaphragm muscle causes the organs of the abdomen to be pushed down and outwards, thereby increasing the available volume of the lungs.

Monitoring and analyzing respiratory characteristics can be particularly useful in athletic applications, as there is a direct link between performance and an athlete's processing of oxygen and carbon dioxide. For example, in many athletic training situations, it is helpful to know when the athlete's body transitions between aerobic exercise and anaerobic exercise, sometimes referred to as the athlete's ventilatory threshold. Crossing over the ventilatory threshold level is an indicator of pending performance limitations during sport activities. For example, it can be beneficial for athletes to train in the anaerobic state for limited periods of time. However, for many sports, proper training requires only limited periods of anaerobic exercise interrupted by lower intensity aerobic exercises. It is difficult for an athlete to determine which state, anaerobic or aerobic, he or she is in without referencing physiological characteristics such as respiratory characteristics. Therefore, respiratory monitoring and data processing can provide substantial benefits in athletic training by allowing for accurate and substantially instantaneous measurements of the athlete's exercise state. Changes in an athlete's ventilatory threshold over time, as well as patterns of tidal volume during post-exercise recovery, can be valuable to measure improvements in the athlete's fitness level over the course of a training regime. Respiratory monitoring can further allow for monitoring and analyzing changes in a subject's resting metabolic rate.

A second ventilatory threshold exists at the point when the load on the body is such that the pulmonary ventilation is no longer sufficient to support life sustainably. Dwelling too long in this state will lead to collapse and so determination of this point can be of value in medical applications, and particularly to first responders and other emergency response personnel.

As indicated above, the present invention is directed to methods of data interpretation and inference making that are possible when a multitude of physiological or performance signals are captured continuously (or pseudo continuously), synchronously, and simultaneously. In some embodiments, context is added to an individual data stream when that data stream is combined with other data streams. For example, when considering heart activity such as, for example, heart rate or pattern, an elevated heart rate can be determined to be a normal or an abnormal condition.

As also stated above, the addition of synchronous respiratory and activity data enables interpretation of the elevated heart rate observation, for example, in a way that would be impossible absent the other signals. As is well known in the art, an elevated heart rate occurring with an increase in respiratory activity and physical activity is a completely normal condition. However, an increase in heart rate and respiratory rate without a concurrent increase in activity indicates physical or emotional stress, and an increase in heart rate without an increase in respiratory rate or activity could signal an impending cardiac arrhythmia or other undesirable sequelae. This sort of interpretive power is only possible when there are multiple signals (or data streams) collected from an individual in a synchronous concurrent manner. Physical activity data can include simply an indication of whether the subject is engaged in physical activity, and if so, the level of physical activity engaged in, and can also include indications of what type of physical activity the subject is engaged in, for example, playing a sport, running, playing basketball, playing baseball, performing push-ups, or walking up stairs.

Applications for this interpretive approach enhance both real-time monitoring and the amount and value of the information in research data from a large number of conditions including acute events (e.g., cardiac arrest, respiratory distress, asthma attack, and allergic reactions), psychological events (e.g., panic/anxiety attacks), chronic disease monitoring (e.g., chronic obstructive pulmonary disease, congestive heart failure, asthma, coronary artery disease, hypertension, diabetes, obesity, depression, and anxiety).

Various physiological monitoring systems and methods can be employed within the scope of the invention to obtain a plurality of signals reflecting physiological, performance, and contextual information.

Several embodiments of preferred physiology monitoring systems and associated methods are described in detail below. The invention, however, is not limited to the systems and associated methods described herein. As will be appreciated by one having ordinary skill in the art, systems and associated methods similar to or equivalent to the described systems and methods can also be employed within the scope of the present invention.

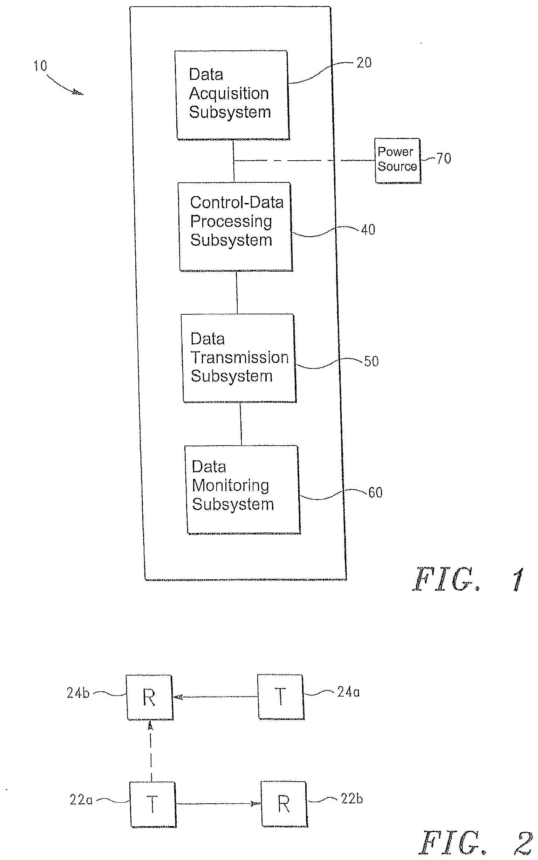

Referring first to Fig. 1, there is shown a schematic illustration of one embodiment of a physiology monitoring system according to the present invention. As illustrated in Fig. 1, the physiology monitoring system 10 preferably includes a data acquisition subsystem 20, a control-data processing subsystem 40, a data transmission subsystem 50, a data monitoring subsystem 60, and a power source 70, such as a battery. Control-data processing subsystem 40 is also referred to herein as "processor subsystem," "processing subsystem," and "data processing subsystem." The terms control-data processing subsystem, processor subsystem, processing subsystem, and data processing subsystem are used interchangeably in the present application.

### Data Acquisition Subsystem

In accordance with one embodiment of the invention, the data acquisition subsystem 20 includes means for acquiring anatomical parameters that can be employed to determine at least one respiratory characteristic, more preferably a plurality of respiratory characteristics, in cooperation with control-data processing subsystem 40, and, in some embodiments, data monitoring subsystem 60. The anatomical parameters may include changes in (or displacements of) the anteroposterior diameters of the rib cage and abdomen, and axial displacement of the chest wall. The means for acquiring the noted parameters, e.g., sensors. The sensors can include paired electromagnetic coils or magnetometers.

Although the present invention is described herein in terms of magnetometers and magnetometer systems, it is understood that other types of sensor systems capable of measuring changes in distance between two or more sensors in the system can be used in place of, or in addition to, magnetometers. Specifically, the invention is not limited to the use of electromagnetic coils or magnetometers to acquire signals representing measured changes in the anteroposterior diameters of the rib cage and abdomen, and axial displacement of the chest wall. Various additional means and devices that can be readily adapted to measure the noted anatomical parameters can be employed within the scope of the invention. Such means and devices include, without limitation, Hall effect sensors and electronic compass sensors. Wireless sensors with the capability of measuring time delay in a signal sent from one sensor to another and thereby determine the distance between the two sensors can be substituted for or provided in addition to magnetometers in accordance with the present invention.

Magnetometers (or other sensors) can be embedded in or carried by a wearable garment, such as a shirt, vest, or sports brassiere. Magnetometers can also be embedded in or carried by a chest strap. The wearable monitoring garment eliminates the need to attach the magnetometers directly to the skin of a subject and, hence, resolves all issues related thereto. The wearable monitoring garment also facilitates repeated and convenient positioning of magnetometers at virtually any appropriate (or desired) position on a subject's torso.

According to the invention, at least one, and preferably two, magnetometers are employed to measure the noted subject parameters (or displacements). In some embodiments of the invention, two pairs of magnetometers are thus employed. In some embodiments, more than two pairs of magnetometers are employed.

Referring now to Fig. 2, there is shown one embodiment of a dual-paired electromagnetic coil arrangement for detecting and measuring displacement(s) of the rib cage, abdomen, and chest wall. As illustrated in Fig. 2, the electromagnetic coils include first transmission coil 22a, first receive coil 22b, second transmission coil 24a, and second receive coil 24b. In Fig. 2, the letter T designates the transmission coils and the letter R designates the receiving coils, however, the coils are not limited to such designations. The electromagnetic coils of embodiments of the present invention are described as "receiving" or "transmitting," however, each receiving coil can alternatively and independently be a transmitting coil, and each transmitting coil can alternatively and independently be a transmitting coil. Coils can also perform both receiving and transmitting functions.

Details of the noted arrangement and associated embodiments (discussed below) are set forth in U.S. Patent Application No. 12/231,692, filed September 5, 2008, U.S. Patent Application No. 61/275,576, filed September 1, 2009, and U.S. Patent Application No. 12/869,576.

As set forth in the noted applications, in some embodiments, at least receive coil 24b is adapted to receive coil transmissions from each of transmission coils 22a, 24a (i.e., at least receive coil 24b may be a dual function coil, where a "dual function coil" refers to a coil capable of receiving transmissions from a plurality of different transmission coils). In some embodiments, each receive coil 22b, 24b is adapted to receive transmissions from each transmission coil 22a, 24a.

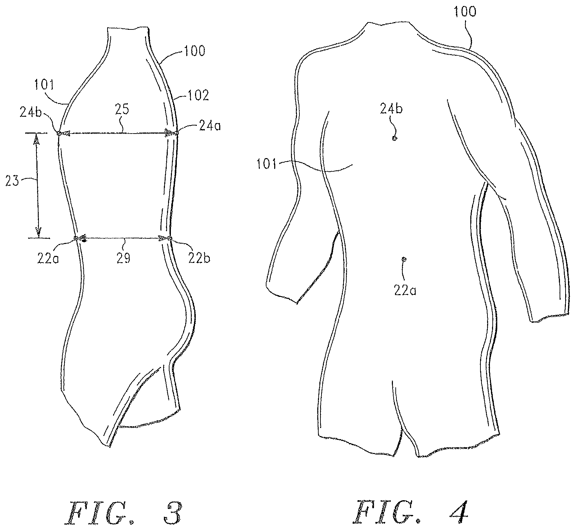

Referring now to Figs. 3-5, there are shown coils 22a, 22b, 24a, 24b positioned on a subject or patient 100. As illustrated in Figs. 3-5, first transmission coil 22a is preferably positioned on front 101 of subject 100 proximate the umbilicus of subject 100, and first receive coil 22b is preferably positioned proximate the same axial position, but on back 102 of subject 100. Second receive coil 24b is preferably positioned on front 101 of subject 100 proximate the base of the sternum and second transmission coil 24a is preferably positioned proximate the same axial position, but on the back 102 of subject 100.

As set forth in co-pending U.S. Patent Application No. 12/231,692, the positions of transmission coils 22a, 24a and receive coils 22b, 24b can be reversed (i.e., transmission coil 22a and receive coil 24b can be placed on back 102 of subject 100 and transmission coil 24a and receive coil 22b can be placed on front 101 of subject 100). Both transmission coils 22a and 24a can also be placed on front 101 or back 102 of subject 100, and receive coils 22b and 24b can be placed on the opposite side.

Referring back to Fig. 3, an arrow 23 represents the chest wall or, in this instance, the xiphi-umbilical distance (Xi) that is monitored. An arrow 25 represents the monitored rib cage distance, while an arrow 29 represents the monitored abdominal distance.

In one embodiment, wherein coil 24b is a dual function coil, as subject or patient 100 breathes, displacement(s) of the rib cage and abdomen (i.e., changes in the distance between each pair of coils 22a, 22b and 24a, 24b, denoted, respectively, by arrow 29 and arrow 25) is determined from measured changes in voltage between paired coils 22a, 22b and 24a, 24b. The axial displacement of the chest wall, denoted by arrow 23 (e.g., xiphiumbilical distance (Xi)) is also determined from measured changes in voltage between transmission coil 22a and receive coil 24b.

As indicated above, more than two pairs of electromagnetic coils can be employed to acquire anatomical parameters. As set forth in U.S. Patent Application No. 61/275,575, filed September 1, 2009, and U.S. Patent Application No. 12/869,582, adding additional electromagnetic coils in anatomically appropriate positions on a subject provides numerous significant advantages over dual-paired coil embodiments. Among the advantages is the provision of additional (and pertinent) data and/or information regarding chest wall movement(s) and the relationship(s) thereof to respiratory activity and respiratory associated events, such as speaking, sneezing, laughing, and coughing.

Further, the multiple single, cross, and interaction axes of the electromagnetic coil transmissions that result from the additional coils (and placement thereof) provide highly accurate quantification of changes in chest wall volume, and facilitate three-dimensional modeling of chest wall shape and movement of ambulatory subjects, and the evaluation and quantification of ventilatory mechanics (e.g., synchronous and asynchronous movement of the chest wall compartments).

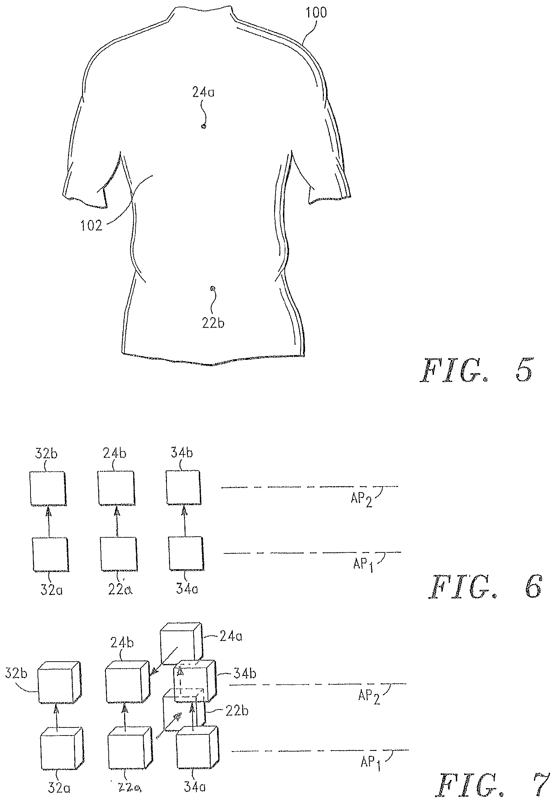

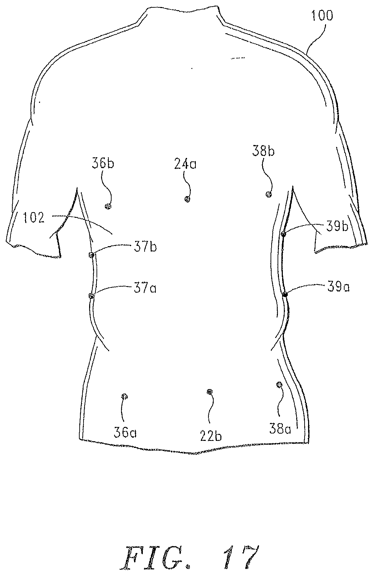

Referring now to Figs. 6-17, several multiple-paired electromagnetic coil arrangements will now be described in detail. It is, however, to be understood that the invention is not limited to the multiple-paired coil embodiments described herein. Indeed, the multiple-paired coil embodiments can comprise any number of additional pairs of electromagnetic coils (e.g., 3, 4, 5, 6, 7, 8, 9, 10, etc.). For example, in embodiments using three magnetometers, for example, electromagnetic coils, it is understood that the three electromagnetic coils can function as multiple pairs. Specifcally, referring to the coils as first, second, and third coils, the first coil can form a pair with the second coil and the first coil can also form a pair with the third coil. In addition, the second coil can also form a pair with the third coil. Thus, a magnetometer system utilizing three electromagnetic coils can be configured to form one, two, or three pairs. Each of the first, second, and third coils can be configured to transmit signals, receive signals, or to both receive and transmit signals. A magnetometer can communicate with a plurality of other magnetometers, and therefore a particular magnetometer can form a part of more than one pair. The position of the additional coils and the function thereof can also be readily modified and/or adapted for a particular application within the scope of the present invention.

Referring first to Figs. 6-8, there is shown one embodiment of a multiple-paired coil arrangement. As illustrated in Fig. 7, the noted embodiment similarly includes electromagnetic coils 22a, 22b, 24a, 24b. According to the invention, any of the aforementioned dual-paired coil embodiments associated with coils 22a, 22b, 24a, 24b can be employed with the multiple-paired coil embodiments of the invention.

As also illustrated in Figs. 6 and 7, the multiple-paired coil arrangement further includes at least two additional pairs of electromagnetic coils: third transmission coil 32a, third receive coil 32b, fourth transmission coil 34a, and fourth receive coil 34b.

In some embodiments, at least one of receive coils 32b, 34b is a dual function coil and, hence, adapted to receive transmissions from each of transmission coils 32a, 22a, 34a. In some embodiments, each receive coil 32b, 34b is adapted to receive transmissions from each transmission coil 32a, 22a, 34a.

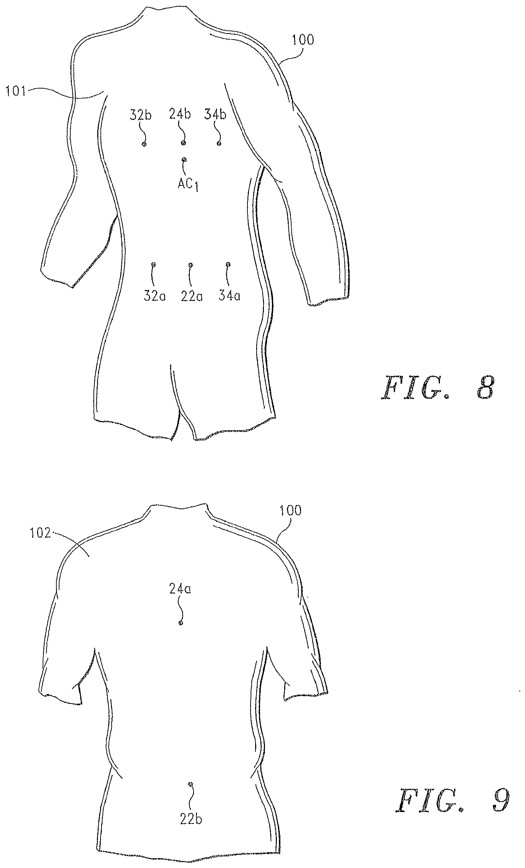

Referring now to Figs. 8 and 9, there are shown coils 22a, 22b, 24a, 24b, 32a, 32b, 34a, 34b positioned on a subject or patient 100. As illustrated in Figs. 8 and 9, first transmission coil 22a is preferably positioned on front 101 of subject 100 proximate the umbilicus of subject 100 and first receive coil 22b is preferably positioned proximate the same axial position, but on back 102 of subject 100. Second receive coil 24b is preferably positioned on front 101 of subject 100 proximate the base of the sternum and second transmission coil 24a is positioned proximate the same axial position, but on back 102 of subject 100.

Third transmission coil 32a is preferably positioned on front 101 of subject 100 and axially spaced to the right of first transmission coil 22a. Fourth transmission coil 34a is preferably positioned on front 101 of subject 100 and axially spaced to the left of first transmission coil 22a. In the illustrated embodiment, each transmission coil 32a, 22a, 34a is preferably positioned proximate the same axial plane (denoted "AP₁" in Figs. 6 and 7).

Third receive coil 32b is preferably positioned on front 101 of subject 100 and axially spaced to the right of second receive coil 24b. Fourth receive coil 34b is preferably positioned on front 101 of subject 100 and axially spaced to the left of second receive coil 24b. Preferably, each receive coil 32b, 24b, 34b is similarly positioned proximate the same axial plane (denoted "AP₂" in Figs. 6 and 7).

The axial spacing of coils 32a, 32b, 34a, 34b will, in many instances, be dependant on the body size and structure of the subject (e.g., adult, female, male, adolescent). The distance between and amongst the coils can also vary with the degree of measurement precision required or desired.

As indicated above, a significant advantage of the multiple-paired coil arrangements is the provision of multiple single, cross, and interaction coil transmission axes that facilitate three-dimensional modeling of chest wall shape and movement of ambulatory subjects, and evaluation and quantification of ventilatory mechanics (e.g., synchronous and asynchronous movement of the chest wall compartments).

A further significant advantage of the multiple-paired coil arrangements is that real-time, three-dimensional models of the chest wall can be created by simultaneous monitoring of the chest wall with the multiple-paired coils.

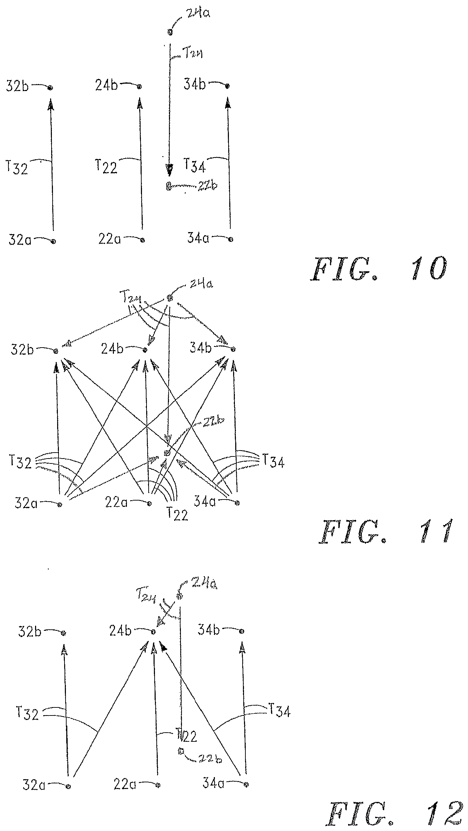

Referring now to Figs. 10-12, there are shown several schematic illustrations of coil transmission axes provided by three multiple-paired coil embodiments of the invention. Referring first to Fig. 10, there is shown an illustration of coil transmissions, wherein each receive coil 32b, 24b, 34b, 22b is a single function coil. Receive coil 32b is adapted to receive a transmission T₃₂ from transmission coil 22a. Receive coil 24b is adapted to receive a transmission T₂₂ from transmission coil 22a. Receive coil 34b is adapted to receive a transmission T₃₄ from transmission coil 34a. Receive coil 22b is adapted to receive a transmission T₂₄ from transmission coil 24a.

Referring now to Fig. 12, there is shown another illustration of coil transmissions, wherein receive coil 24b is a dual function coil. Receive coil 32b is adapted to receive transmission T₃₂ from transmission coil 32a, receive coil 34b is adapted to receive transmission T₃₄ from transmission coil 34a, and receive coil 22b is adapted to receive transmission T₂₄ from transmission coil 24a. Receive coil 24b is, however, adapted to receive transmission T₃₂ from transmission coil 32a, transmission T₂₂ from transmission coil 22a, transmission T₃₄ from transmission coil 34a, and transmission T₂₄ from transmission coil 24a.

In Fig. 11, each receive coil 32b, 24b, 34b, 22b is a dual function coil. As illustrated in Fig. 11, receive coil 32b is thus adapted to receive transmission T₃₂ from transmission coil 32a, transmission T₂₂ from transmission coil 22a, transmission T₃₄ from transmission coil 34a, and transmission T₂₄ from transmission coil 24a. Receive coils 24b, 34b, and 22b are also adapted to receive transmission T₃₂ from transmission coil 32a, transmission T₂₂ from transmission coil 22a, transmission T₃₄ from transmission coil 34a, and transmission T₂₄ from transmission coil 24a.

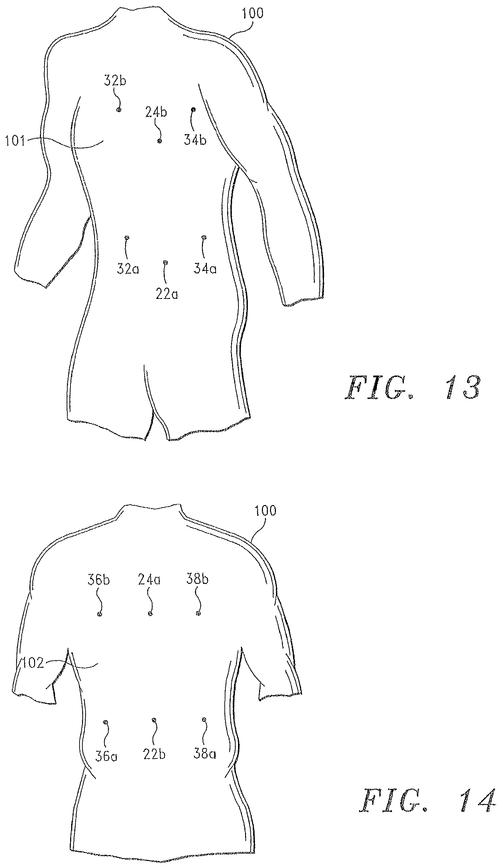

Referring now to Figs. 13-17, there are shown additional multiple-paired coil arrangements that can be employed within the scope of the present invention. Referring first to Fig. 13, there is shown a multiple-paired coil arrangement, wherein the two additional coil pairs 32a, 32b and 34a, 34b are non-uniformly positioned on front 101 of subject 100. As indicated, the additional coils can be positioned at any appropriate (or desired) positions on the torso of subject 100.

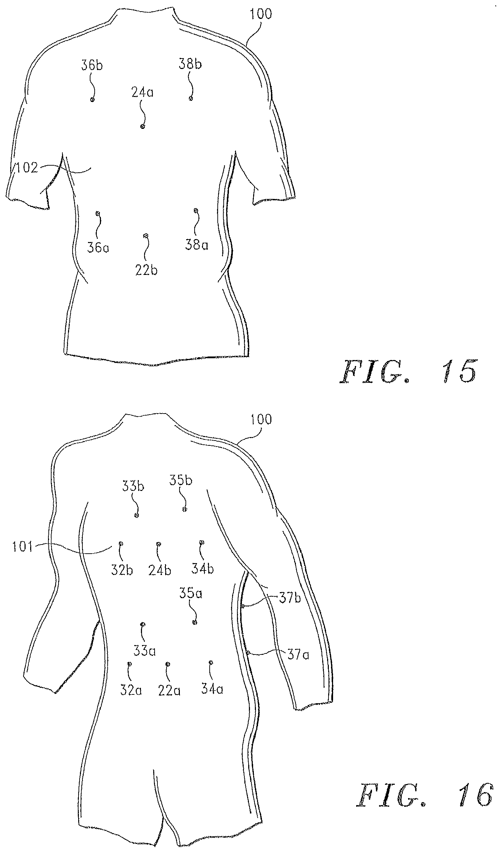

Additional coils (e.g., transmission coil 36a paired with receive coil 36b, and transmission coil 38a paired with receive coil 38b) can also be positioned on back 102 of subject 100, as illustrated in Fig. 14. Coils 36a, 36b, 38a, 38b can be positioned uniformly, as shown in Fig. 14, or non-uniformly, as illustrated in Fig. 15.

Referring now to Figs. 16-17, there is shown another multiple-paired coil arrangement, wherein additional coils are positioned on the torso of subject 100. As illustrated in Fig. 16, additional coils (e.g., transmission coil 33a paired with receive coil 33b, and transmission coil 35a paired with receive coil 35b) can be positioned on front 101 of subject 100. Additional coils (e.g., transmission coil 37a paired with receive coil 37b, and transmission coil 39a paired with receive coil 39b) can be positioned on opposite sides of subject 100.

Additionally, the transmission coils and receive coils disclosed herein need not necessarily be paired one-to-one. For example, a single receive coil may be configured to receive transmissions from multiple transmission coils, and a single transmission coil may be configured to transmit to multiple receive coils.

Multiple coil embodiments of the invention are not limited to the multiple-paired coil embodiments shown in Figs. 6-17. It is again emphasized that the multiple-paired coil embodiments can include any number of additional electromagnetic coils. Further, the position of the additional coils and the function thereof can also be readily modified and/or adapted for a particular application within the scope of the present invention.

Data acquisition subsystem 20 can also include means for directly monitoring the orientation and/or movement of subject 100 (e.g., spatial parameters). Such means can include optical encoders, proximity and Hall effect switches, laser interferometry, accelerometers, gyroscopes, and/or global positioning systems (GPS). The data obtained from such means may provide performance parameters or characteristics of a subject engaged in physical activity, such as, for example, speed or acceleration of the subject or a portion of the subject, pace, stride length, stride count and repetition count.

In one embodiment, the means for directly monitoring the orientation and movement of a subject includes at least one multi-function inertial sensor (e.g., 3-axis accelerometer or 3-axis gyroscope). As is well known in the art, orientation and motion of a subject can be readily determined from the signals or data transmitted by a multi-function inertial sensor.

According to the present invention, the accelerometer can be disposed in any anatomically appropriate position on subject 100. In one embodiment of the invention, an accelerometer (denoted "AC1" in Fig. 8) is disposed proximate the base of the sternum of subject 100.

In some embodiments, multiple accelerometers (or other means for monitoring orientation and/or movement of subject 100) may be positioned at various locations on the body of subject 100. Such positioning may allow for acquisition of data relating to motion of portions of the body of subject 100 relative to other portions of the body of subject 100, as well as relative to the environment.

In some embodiments, performance parameters including mental or emotional parameters such as, for example, stress or motivation level may be determined. Indications of such parameters can include, for example, trunk angle or foot strike characteristics, and can be monitored by the above-described means for monitoring orientation and/or movement of subject 100.

Data acquisition subsystem 20 can additionally include at least one additional physiological sensor (preferably a plurality of additional physiological sensors) adapted to monitor and record one or more physiological characteristics associated with monitored subject 100. The physiological sensors can include, without limitation, sensors that are adapted to monitor and record electrical activity of the brain, heart, and other muscles (e.g., EEG, ECG, EMG), pulse rate, blood oxygen saturation level (e.g., SpO₂), skin temperature, and core temperature. Physiological parameters measured and/or calculated may include, for example, heart rate, respiration rate, blood oxygen level, blood flow, hydration status, calories burned, muscle fatigue, and/or body temperature.

The additional sensors can, of course, be disposed in a variety of anatomically appropriate positions on a subject. By way of example, a first sensor (e.g., a pulse rate sensor) can be disposed proximate the heart of subject 100 to monitor pulse rate, and a second sensor (e.g., a microphone) can be disposed proximate the throat of subject 100 to monitor sounds emanating therefrom (e.g., sounds reflecting coughing).

Data acquisition subsystem 20 can also include one or more audio sensors, such as a microphone for monitoring sounds generated by monitored subject 100, and a speaker, to enable two-way communication by and between monitored subject 100 and a monitoring station or individual, such as, for example, an athletic trainer or medical personnel.

According to exemplary embodiments of the invention, the paired coils (e.g., electromagnetic coils 22a, 22b and 24a, 24b) and the aforementioned additional sensors can be positioned on or proximate subject 100 by various suitable means. Thus, the paired coils and/or additional sensors can, for example, be directly attached to subject 100 and/or can be included in a garment (e.g., athletic apparel) or accessory (e.g., watch, belt) worn by subject 100.

As set forth in U.S. Patent Application No. 61/275,633, filed September 1, 2009, and U.S. Patent Application No. 12/869,627, the paired coils, additional sensors, processing and monitoring systems (e.g., local data units (LDUs) if employed), and associated wiring, cabling, and other power and signal transmission apparatuses and/or systems can also be embedded in or carried by a wearable garment or item that can be comfortably worn by a monitored subject.

U.S. Patent Application No. 61/275,576, filed September 1, 2009, and U.S. Patent Application No. 12/869,576, also disclose magnetometer-based wearable monitoring garments.

### Control-Data Processing Subsystem

Control-data processing subsystem 40 includes programs, instructions, and associated algorithms and parameters to control data acquisition subsystem 20 and, hence, the paired electromagnetic coils (e.g., coils 22a, 22b, 24a, 24b, 32a, 32b, 34a, 34b) and the function thereof, and the transmission and receipt of coil transmissions (e.g., transmissions T₃₂, T₂₂, T₃₄, and T₂₄) as well as data transmission subsystem 50 and data monitoring subsystem 60. Such is discussed in detail below.

Control-data processing subsystem 40 is further programmed and adapted to retrieve and process coil transmissions or signals from the electromagnetic coils (e.g., coils 22a, 22b, 24a, 24b, 32a, 32b, 34a, 34b) in order to determine physiological information associated with monitored subject 100, to retrieve, process, and interpret additional signals transmitted by additional spatial parameter and physiological sensors (discussed below), and to transmit selective coil data, physiological and spatial parameters, physiological characteristics, and subject information to data monitoring subsystem 60 of the invention.

Control-data processing subsystem 40 further includes at least one "n-degrees-of-freedom" model or algorithm for determining at least one respiratory characteristic (e.g., V_{T}) from the retrieved coil transmissions or signals (e.g., measured displacements of the rib cage, abdomen, and chest wall).

Control-data processing subsystem 40 also preferably includes suitable algorithms that are designed and adapted to conduct multivariable analyses of data acquired by data acquisition subsystem 20 (e.g., coil transmissions and signals transmitted by additional spatial parameter and physiological sensors, as discussed below).

Control-data processing subsystem 40 also preferably includes suitable algorithms that are designed and adapted to determine respiratory characteristics, parameters, and statuses from measured multiple, interactive chest wall displacements. The algorithms are also preferably adapted to discount measured chest wall displacements that are associated with non-respiration movement (e.g., twisting of the torso) to enhance the accuracy of respiratory characteristic (and/or parameter) determinations.

Control-data processing subsystem 40 additionally preferably includes suitable programs, algorithms, and instructions to generate three-dimensional models of the chest wall of subject 100 from the measured multiple, interactive chest wall displacements.

Control-data processing subsystem 40 is also preferably programmed and adapted to determine additional and, in some instances, interrelated anatomical parameters, such as bending, twisting, coughing, etc., from the measured multiple, interactive chest wall displacements. In some instances, control-data processing subsystem 40 is programmed and adapted to compare retrieved coil transmissions reflecting measured chest wall displacements with stored selective combinations of coil transmissions and chest wall parameters that are associated therewith (e.g., "normal respiration and bending", "normal respiration and coughing").

By way of example, in one embodiment, a first chest wall parameter (CWP₁) defined as (or reflecting) "normal respiration and twisting of the torso" is stored in control-data processing subsystem 40. The coil transmissions and data associated with the first chest wall parameter (CWP₁) include transmissions T₃₂, T₂₂, T₃₄, and T₂₄ received by receive coil 24b that can represent displacements x, y, and z.

During monitoring of subject 100, similar coil transmissions may be received by receive coil 24b. Control-data processing subsystem 40 then compares the detected (or retrieved) transmissions to the stored transmissions and chest wall parameters associated therewith to determine (in real-time) the chest wall movement and, hence, respiratory activity based thereon; in this instance "normal respiration and twisting of the torso".

In some embodiments, the signals transmitted by the accelerometer (e.g., spatial parameter signals) are employed with the detected coil transmissions to determine and classify chest wall movement and associated respiratory activity of the monitored subject. In the noted embodiments, each stored chest wall parameter also includes spatial parameter signals associated with the chest wall parameter (e.g., normal respiration and twisting of the torso). Control-data processing subsystem 40 is adapted to compare retrieved coil transmissions and spatial parameter signals to the stored transmissions and spatial parameter signals, and the chest wall parameters associated therewith, to determine the chest wall movement and, hence, respiratory activity based thereon.

In some instances, control-data processing subsystem 40 is programmed and adapted to determine chest wall movement and respiratory activity based on retrieved coil transmissions, spatial parameter signals, and audio signals. In the noted embodiments, data acquisition subsystem 20 may also include an audio sensor, such as, for example, a microphone, that is disposed in an anatomically appropriate position on subject 100 (e.g., proximate the throat).

In this instance, each stored chest wall parameter also includes at least one audio parameter (e.g., > N db, based on the audio signal) that is associated with the chest wall parameter (e.g., normal respiration and coughing). Upon receipt of coil transmissions, spatial parameter signals, and audio signals, control-data processing subsystem 40 compares the retrieved coil transmissions, spatial parameter signals, and audio signals to the stored transmissions, spatial parameter signals, and audio parameters, and the chest wall parameters associated therewith, to determine the chest wall movement and respiratory activity based thereon (e.g., normal respiration and coughing).

### Data Monitoring Subsystem

Data monitoring subsystem 60 is designed and adapted to receive and, in some embodiments, selectively monitor coil transmissions or signals (e.g., transmissions T₃₂, T₂₂, T₃₄, and T₂₄) and to display parameters associated therewith (e.g., displacement(s) along a selective axis), and/or to display a chest wall parameter (e.g., CWP₁), and/or to display a respiratory characteristic (e.g., V_{T}) or event. Such display can be via a variety of media, such as a personal digital assistant (PDA), a mobile phone, and/or a computer monitor, etc.

Data monitoring subsystem 60 is further preferably designed and adapted to display selective subject parameters, characteristics, information, and warnings or alarms. The data monitoring subsystem can also be adapted to display or broadcast data aurally. The aurally presented data can be voice messages, music, or other noises signifying an event. The data monitoring subsystem can be adapted to allow headphones or speakers to connect to the data monitoring subsystem, either wireless or wired, to display the aural data.

In some instances, data monitoring subsystem 60 is also adapted to receive and, in some embodiments, selectively monitor spatial parameter signals and signals transmitted by additional anatomical and physiological sensors (e.g., signals indicating skin temperature or SpO₂) and to display parameters and information associated therewith. The parameters can be associated with an athlete's physical activity. Physical or anatomical parameters measured and/or calculated may include, for example, time, location, distance, speed, pace, stride count, stride length, stride rate, and/or elevation. Physiological parameters measured and/or calculated may include, for example, heart rate, respiration rate, blood oxygen level, blood flow, hydration status, calories burned, muscle fatigue, and/or body temperature. In an embodiment, performance parameters may also include mental or emotional parameters or activity such as, for example, stress level or motivation level. Mental and emotional parameters or activity may be measured and/or calculated directly or indirectly either through posing questions to the athlete or by measuring things such as, for example, trunk angle or foot strike characteristics while running.

In some embodiments, data monitoring subsystem 60 includes a local electronic module or local data unit (LDU). By the term "local" as used in connection with a LDU, it is meant that the LDU is disposed close to the electromagnetic coils, such as on or in a wearable garment containing the coils (which is discussed in detail below). The LDU is preferably adapted to receive and monitor coil transmissions (or signals), preprocess the coil transmissions, and store the coil transmissions and related data.

In some embodiments, the LDU is also adapted to receive and monitor the spatial parameter transmissions (or signals) and additional signals transmitted by additional anatomical and physiological sensors (if employed), to preprocess the signals, and to store the signals and related data.

### Data Transmission Subsystem

Data transmission subsystem 50 includes communication protocols to (i) transmit control signals to data acquisition subsystem 20, (ii) transmit coil transmissions (or signals) from the coils to control-data processing subsystem 40, and (iii) transmit data and information, including coil transmissions (or signals) and related parameters, physiological characteristics, spatial parameters, and subject information from control-data processing subsystem 40 to data monitoring subsystem 60.

In some embodiments, the communication link between data acquisition subsystem 20 and control-data processing subsystem 40 includes conductive wires or similar direct communication means. In some embodiments, the communication link between data acquisition subsystem 20 and control-data processing subsystem 40, as well as between control-data processing subsystem 40 and data monitoring subsystem 60, includes a wireless (or radio) link. The communication link between data acquisition subsystem 20 and control-data processing subsystem 40, and the communication link between control-data processing subsystem 40 and data monitoring subsystem 60 need not be the same type of link.

Data acquisition subsystem 20 can additionally include at least one additional physiological sensor (preferably a plurality of additional physiological sensors) adapted to monitor and record one or more physiological characteristics associated with monitored subject 100. The physiological sensors can include, without limitation, sensors that are adapted to monitor and record electrical activity of the brain, heart, and other muscles (e.g., EEG, ECG, EMG), pulse rate, blood oxygen saturation level (e.g., SpO₂), skin temperature, and core temperature. Physiological parameters measured and/or calculated may include, for example, heart rate, respiration rate, blood oxygen level, blood flow, hydration status, calories burned, muscle fatigue, and/or body temperature.

The additional sensors can, of course, be disposed in a variety of anatomically appropriate positions on a subject. By way of example, a first sensor (e.g., a pulse rate sensor) can be disposed proximate the heart of subject 100 to monitor pulse rate, and a second sensor (e.g., a microphone) can be disposed proximate the throat of subject 100 to monitor sounds emanating therefrom (e.g., sounds reflecting coughing).

Data acquisition subsystem 20 can also include one or more audio sensors, such as a microphone for monitoring sounds generated by monitored subject 100, and a speaker, to enable two-way communication by and between monitored subject 100 and a monitoring station or individual, such as, for example, an athletic trainer or medical personnel.

According to exemplary embodiments of the invention, the paired coils (e.g., electromagnetic coils 22a, 22b and 24a, 24b) and the aforementioned additional sensors can be positioned on or proximate subject 100 by various suitable means. Thus, the paired coils and/or additional sensors can, for example, be directly attached to subject 100 and/or can be included in a garment (e.g., athletic apparel) or accessory (e.g., watch, belt) worn by subject 100.

As set forth in U.S. Patent Application No. 61/275,633, filed September 1, 2009, and U.S. Patent Application No. 12/869,627, the paired coils, additional sensors, processing and monitoring systems (e.g., local data units (LDUs) if employed), and associated wiring, cabling, and other power and signal transmission apparatuses and/or systems can also be embedded in or carried by a wearable garment or item that can be comfortably worn by a monitored subject.

U.S. Patent Application No. 61/275,576, filed September 1, 2009, and U.S. Patent Application No. 12/869,576, also disclose magnetometer-based wearable monitoring garments.

The physiological monitoring systems and methods described above can readily provide a multitude of signals representing physiological, performance, and contextual parameters continuously (or pseudo-continuously), synchronously, and simultaneously.

According to an exemplary embodiment of the present invention, signals and data streams associated with physiological or performance parameters of subject 100 may be received by a processor subsystem, where the processor subsystem may include the above-mentioned systems and subsystems, or portions thereof, or where the processor subsystem may be separate and distinct therefrom. The processor subsystem may interpret the received signals and/or data streams continuously, synchronously, and simultaneously, and may compare signals and data streams associated with different physiological or performance parameters of subject 100.

In comparing and interpreting the data streams representing different physiological or performance parameters continuously, synchronously, and simultaneously, the processor subsystem can determine trends and correlations among the data streams, and can further make inferences as to the subject's level of physical or emotional activity, or distress. Using this data in combination with, for example, historical data, data of other subjects, or appropriate algorithms, the processor subsystem can also determine if the data interpreted from the data streams indicates a contextual parameter about subject 100, such as, for example, presence or absence of a disease or other medical condition, response to treatment, potentiality of an impending medical condition or attack, level of physical fitness, or change in level of physical fitness.

Because the processor subsystem analyzes the plurality of data streams continuously, synchronously, and simultaneously, each data stream is considered in context with the other received data streams. This provides a plurality of reference points for making the above determinations and inferences, and consequently makes the resulting determinations and inferences much more accurate and dependable than would be achieved otherwise.

Acquiring multiple signals continuously (or pseudo-continuously), synchronously, and simultaneously; provides context that enables the interpretation and analysis of data to be more effective. The multivariable analyses capable with this kind of data-set increases the information content of a data-set substantially when compared to any individual data stream on its own.

A significant advantage of the invention is that physiological system functions and/or disease symptoms, particularly chronic disease symptoms, can be rapidly detected and assessed, and preventive treatment promptly initiated or intensified, in order to prevent progression and worsening of a clinical episode and to stop and reverse the pathophysiological process. The invention also facilitates rapid detection and assessment of acute events, such as cardiac arrest, respiratory distress, asthma attack, and allergic reactions, and psychological events, such as panic/anxiety attacks.

Another significant advantage of the invention is that athletic performance characteristics can be detected and assessed, and training programs promptly initiated or intensified, in order to effectively manage a subject's physical fitness. For example, an athlete can pre-set a desired intensity for a workout, and the various data streams can be analyzed in order to provide feedback to the athlete regarding his or her current physical state in relation to the desired intensity. The data streams can include a speed or motion signal from an accelerometer mounted on the athlete, a heart rate signal representing the current heart rate of the athlete, and a respiratory signal representing the athlete's current respiratory condition, e.g., the athlete's tidal volume or respiratory rate as determined by magnetometers mounted on the athlete or attached to a garment worn by the athlete. Comparing the information contained in these three signals continuously, synchronously and/or simultaneously can allow the system to provide more useful feedback to the athlete. For example, by performing a synchronous comparison of a respiratory signal and an accelerometer signal containing information relating to the motion of the athlete, the system can provide feedback regarding the relation of the athlete's motion and the athlete's respiratory characteristics. If, for example, the athlete's respiratory rate indicates that the athlete's respiration is not being strained relative to the motion of the athlete at a given time, the athlete can be encouraged to intensify their workout.

## Claims

1. A system (10) for interpretation and analysis of information relating to a subject (100), the system comprising:
a data acquisition subsystem (20) configured to continuously, synchronously, and simultaneously generate data streams representing parameters of the subject (100); and
a processor subsystem (40) in communication with the data acquisition subsystem (20) and configured to receive the data streams continuously, synchronously, and simultaneously, wherein the processor subsystem (40) is configured to determine a correlation between information contained in the received data streams,
the data acquisition subsystem (20) includes at least a first sensor (22a, 24a) and a second sensor (22b, 24b), wherein the first (22a, 24a) and second (22b, 24b) sensors are responsive to changes in distance therebetween, and wherein the first (22a, 24a) and second (22b, 24b) sensors are configured to be positioned proximate to the subject's body, **characterized in that**
the processor subsystem (40) is further configured to use data of other subjects to determine if the data interpreted from the data streams indicates a contextual parameter about the subject (100), such as presence or absence of a disease or other medical condition, response to treatment, potentiality of an impending medical condition or attack, level of physical fitness, or change in level of physical fitness.

2. The system (10) of claim 1, wherein one of the data streams represents a respiratory parameter, and wherein one of the data streams represents activity data.

3. The system (10) of claim 2, wherein the processor subsystem (40) is further configured to compare the data stream representing the respiratory parameter and the data stream representing activity data synchronously and to determine a condition of the subject (100) based on the comparison.

4. The system (10) of claim 3,
wherein one of the data streams represents heart activity,
wherein the processor subsystem (40) is further configured to determine whether the data stream representing heart activity indicates an elevated heart rate of the subject, and to compare the data stream representing the respiratory parameter, the data stream representing activity data, and the data stream representing heart activity synchronously, and
wherein the processor subsystem (40) is further configured to, in response to a determination that the data stream representing heart activity indicates an elevated heart rate, determine whether the elevated heart rate coincides with one of an increase in respiratory activity indicated by the data stream representing the respiratory parameter and an increase in physical activity indicated by the data stream representing activity data.

5. The system (10) of claim 4, wherein the processor subsystem (40) is further configured to:
determine that the elevated heart rate is a normal condition in response to a determination that the elevated heart rate coincides with one of an increase in respiratory activity and an increase in physical activity, and
determine that the elevated heart rate is an abnormal condition, in response to a determination that the elevated heart rate does not coincide with one of an increase in respiratory activity and an increase in physical activity.

6. The system (10) of claim 1, wherein the system further comprises a garment adapted to cover at least a portion of the subject's chest.

7. The system (10) of claim 6, wherein the first (22a, 24a) and second (22b, 24b) sensors are proximate to the subject's chest region when the garment is worn by the subject.

8. The system (10) of claim 1, wherein the first (22a, 24a) and second (22b, 24b) sensors are electromagnetic coils.

9. The system (10) of claim 8, wherein the data stream representing the respiratory parameter corresponds to signals generated by the electromagnetic coils, the signals being indicative of the change in distance therebetween.

10. A method for interpretation and analysis of information relating to a subject (100), the method comprising:
generating data streams representing parameters of a subject continuously, synchronously, and simultaneously;
receiving the data streams continuously, synchronously, and simultaneously;
determining a contextual parameter from the received plurality of data streams, wherein the data streams are generated by sensors (22a, 22b; 24a; 24b) positioned proximate to the subject's body, **characterized in that**
the step of determining a contextual parameter comprises using data of other subjects to determine if the data interpreted from the data streams indicates a contextual parameter about the subject (100), such as presence or absence of a disease or other medical condition, response to treatment, potentiality of an impending medical condition or attack, level of physical fitness, or change in level of physical fitness.

11. The method of claim 10, wherein one of the data streams represents a respiratory parameter, and wherein one of the data streams represents activity data.

12. The method of claim 11, further comprising:
comparing the data stream representing the respiratory parameter and the data stream representing activity data synchronously; and
determining a condition of the subject based on the comparison.

13. The method of claim 12, further comprising:
determining whether a data stream representing heart activity indicates an elevated heart rate of the subject (100);
comparing the data stream representing the respiratory parameter, the data stream representing activity data, and the data stream representing heart activity synchronously;
determining, in response to a determination that the data stream representing heart activity indicates an elevated heart rate, whether the elevated heart rate coincides with one of an increase in respiratory activity indicated by the data stream representing the respiratory parameter and an increase in physical activity indicated by the data stream representing activity data.

14. The method of claim 13, further comprising:
determining, in response to a determination that the elevated heart rate coincides with one of an increase in respiratory activity and an increase in physical activity,
that the elevated heart rate is a normal condition; and
determining, in response to a determination that the elevated heart rate does not coincide with one of an increase in respiratory activity and an increase in physical activity, that the elevated heart rate is an abnormal condition.

## Patentansprüche

1. Ein System (10) für die Interpretation und Analyse von Informationen, welche sich auf ein Subjekt (100) beziehen, wobei das System umfasst:
ein Datenerfassungssubsystem (20) dazu eingerichtet, um kontinuierlich, synchron und simultan Datenströme zu erzeugen, welche Parameter des Subjekts (100) repräsentieren; und
ein Prozessorsubsystem (40) in Kommunikation mit dem Datenerfassungssubsystem (20) und dazu eingerichtet, um die Datenströme kontinuierlich, synchron und simultan zu empfangen, wobei das Prozessorsubsystem (40) dazu eingerichtet ist, um eine Korrelation zwischen Informationen zu bestimmen, welche in den empfangenen Datenströmen enthalten sind,
wobei das Datenerfassungssubsystem (20) zumindest einen ersten Sensor (22a, 24a) und einen zweiten Sensor (22b, 24b) beinhaltet, wobei der erste (22a, 24a) und zweite (22b, 24b) Sensor auf Distanzänderungen dazwischen reaktionsfähig sind, und wobei der erste (22a, 24a) und zweite (22b, 24b) Sensor dazu eingerichtet sind, um unmittelbar am Körper des Subjekts positioniert zu werden, **dadurch gekennzeichnet, dass**
das Prozessorsubsystem (40) weiter dazu eingerichtet ist, um Daten von anderen Subjekten zu verwenden, um zu bestimmen, ob die Daten, welche von den Datenströmen interpretiert wurden, einen kontextabhängigen Parameter über das Subjekt (100) andeuten, wie etwa Präsenz oder Abwesenheit einer Krankheit oder anderen medizinischen Zustand, Behandlungserfolg, Möglichkeit eines drohenden medizinischen Zustands oder Attacke, Level von physischer Fitness oder Veränderung im Level der physischen Fitness.

2. Das System (10) nach Anspruch 1, wobei einer der Datenströme einen respiratorischen Parameter repräsentiert und wobei einer der Datenströme Aktivitätsdaten repräsentiert.

3. Das System (10) nach Anspruch 2, wobei das Prozessorsubsystem (40) weiter dazu eingerichtet ist, um den Datenstrom, welcher den respiratorischen Parameter repräsentiert, und den Datenstrom, welcher Aktivitätsdaten repräsentiert, synchron zu vergleichen und dazu eingerichtet ist, um einen Zustand des Subjekts (100) basierend auf dem Vergleich zu bestimmen.

4. Das System (10) nach Anspruch 3,
wobei einer der Datenströme Herzaktivität repräsentiert,
wobei das Prozessorsubsystem (40) weiter dazu eingerichtet ist, um zu bestimmen, ob der Datenstrom, welcher Herzaktivität repräsentiert, eine erhöhte Herzrate des Subjekts andeutet und um den Datenstrom, welcher den respiratorischen Parameter repräsentiert, den Datenstrom, welcher Aktivitätsdaten repräsentiert, und den Datenstrom, welcher Herzaktivität repräsentiert, synchron zu vergleichen, und
wobei das Prozessorsubsystem (40) weiter dazu eingerichtet ist, um zu bestimmen, in Antwort zu einer Bestimmung, dass der Datenstrom, welcher Herzaktivität repräsentiert, eine erhöhte Herzrate andeutet, ob die erhöhte Herzrate mit einem der Folgenden übereinstimmt: eine Erhöhung in der respiratorischen Aktivität, welche durch den Datenstrom angedeutet ist, welcher den respiratorischen Parameter andeutet, und eine Erhöhung in der physischen Aktivität, welche durch den Datenstrom angedeutet wird, welcher Aktivitätsdaten repräsentiert.

5. Das System (10) nach Anspruch 4, wobei das Prozessorsubsystem (40) weiter dazu eingerichtet ist, um
zu bestimmen, dass die erhöhte Herzrate ein normaler Zustand ist in Antwort zu einer Bestimmung, dass die erhöhte Herzrate mit einem von einer Erhöhung in der respiratorischen Aktivität und einer Erhöhung in der physischen Aktivität übereinstimmt, und um
zu bestimmen, dass die erhöhte Herzrate ein abnormaler Zustand ist in Antwort zu einer Bestimmung, dass die erhöhte Herzrate nicht mit einem einer Erhöhung in der respiratorischen Aktivität und einer Erhöhung in der physischen Aktivität übereinstimmt.

6. Das System (10) nach Anspruch 1, wobei das System weiter ein Kleidungsstück umfasst, welches dazu geeignet ist, um zumindest einen Teil der Brust des Subjekts zu bedecken.

7. Das System (10) nach Anspruch 6, wobei der erste (22a, 24a) und zweite (22b, 24b) Sensor unmittelbar in der Brustregion des Subjekts sind, wenn das Kleidungsstück durch das Subjekt getragen wird.

8. Das System (10) nach Anspruch 1, wobei der erste (22a, 24a) und zweite (22b, 24b) Sensor elektromagnetische Spulen sind.

9. Das System (10) nach Anspruch 8, wobei der Datenstrom, welcher den respiratorischen Parameter repräsentiert, Signalen entspricht, welche durch die elektromagnetischen Spulen erzeugt wurden, wobei die Signale indikativ auf Distanzänderungen dazwischen sind.

10. Ein Verfahren zur Interpretation und Analyse von Informationen, welche zu einem Subjekt (100) sich beziehen, wobei das Verfahren umfasst:
kontinuierliches, synchrones und simultanes Erzeugen von Datenströmen, welche Parameter eines Subjekts repräsentieren;
kontinuierliches, synchrones und simultanes Empfangen von Datenströmen;
Bestimmen eines kontextabhängen Parameters von der empfangenen Vielzahl von Datenströmen, wobei die Datenströme erzeugt werden durch Sensoren (22a, 22b; 24a; 24b), welche unmittelbar am Körper des Objektes positioniert sind, **dadurch gekennzeichnet, dass**
der Schritt des Bestimmens eines kontextabhängen Parameters Verwenden von Daten von anderen Subjekten umfasst, um zu bestimmen, ob die Daten, welche von Datenströmen interpretiert wurden, einen kontextabhängigen Parameter über das Subjekt (100) andeuten, wie etwa Präsenz oder Abwesenheit einer Krankheit oder anderen medizinischen Zustand, Behandlungserfolg, Möglichkeit eines drohenden medizinischen Zustands oder Attacke, Level von physischer Fitness oder Änderung der Level der physischen Fitness.

11. Das Verfahren nach Anspruch 10, wobei einer der Datenströme einen respiratorischen Parameter repräsentiert und wobei einer der Datenströme Aktivitätsdaten repräsentiert.

12. Das Verfahren nach Anspruch 11, weiter umfassend:
synchrones Vergleichen des Datenstroms, welcher den respiratorischen Parameter repräsentiert, und des Datenstroms, welcher Aktivitätsdaten repräsentiert; und
Bestimmen eines Zustands des Subjekts basierend auf dem Vergleich.

13. Das Verfahren nach Anspruch 12, weiter umfassend;
Bestimmen, ob ein Datenstrom, welcher Herzaktivität repräsentiert, eine erhöhte Herzrate des Subjekts (100) andeutet;
synchrones Vergleichen des Datenstroms, welcher den respiratorischen Parameter repräsentiert, des Datenstroms, welcher Aktivitätsdaten repräsentiert, und des Datenstroms, welcher Herzaktivität repräsentiert;
Bestimmen, in Antwort zu einer Bestimmung, dass der Datenstrom, welcher Herzaktivität repräsentiert, eine erhöhte Herzrate andeutet, ob die erhöhte Herzrate mit einer Erhöhung in der respiratorischen Aktivität, welche durch den Datenstrom angedeutet wird, welcher den respiratorischen Parameter repräsentiert, und einer Erhöhung in der physischen Aktivität, welche durch den Datenstrom, welcher durch Aktivitätsdaten repräsentiert wird, angedeutet wird.

14. Das Verfahren nach Anspruch 13, weiter umfassend:
Bestimmen, in Antwort zu einer Bestimmung, dass die erhöhte Herzrate mit einem einer Erhöhung in der respiratorischen Aktivität und einer Erhöhung in der physischen Aktivität übereinstimmt, dass die erhöhte Herzrate ein normaler Zustand ist; und
Bestimmen, in Antwort zu einer Bestimmung, dass die erhöhte Herzrate nicht mit einem einer Erhöhung in der respiratorischen Aktivität und einer Erhöhung in der physischen Aktivität übereinstimmt, dass die erhöhte Herzrate ein abnormaler Zustand ist.

## Revendications

1. Un système (10) pour l'interprétation et l'analyse d'informations concernant un patient (100), le système comprenant :
un sous-système d'acquisition de données (20) configuré pour générer de façon continue, synchrone et simultanée des flux de données représentant des paramètres du patient (100) ; et
un sous-système processeur (40) en communication avec le sous-système d'acquisition de données (20) et configuré pour recevoir les flux de données de manière continue, synchrone et simultanée, le sous-système processeur (40) étant configuré pour déterminer une corrélation entre informations contenues dans les flux de données reçus,
le sous-système d'acquisition de données (20) incluant au moins un premier capteur (22a, 24a) et un second capteur (22b 24b), le premier (22a, 24a) et le second (22b, 24b) capteur étant sensibles à des modifications de la distance entre eux, et le premier (22a, 24a) et le second (22b, 24b) capteur étant configurés pour être positionnés à proximité du corps du patient,
**caractérisé en ce que**
le sous-système processeur (40) est en outre configuré pour utiliser les données d'autres patients pour déterminer si les données interprétées à partir des flux de données indiquent un paramètre contextuel relatif au patient (100), tel que la présence ou l'absence d'une pathologie ou autre état médical, une réponse à un traitement, une potentialité de crise ou d'état médical imminents, un niveau de forme physique, ou une modification du niveau de forme physique.

2. Le système (10) de la revendication 1, dans lequel l'un des flux de données représente un paramètre respiratoire, et dans lequel l'un des flux de données représente des données d'activité.

3. Le système (10) de la revendication 1, dans lequel le sous-système processeur (40) est en outre configuré pour comparer le flux de données représentant le paramètre respiratoire au flux de données représentant les données d'activité de façon synchrone et pour déterminer un état du patient (100) sur la base de la comparaison.

4. Le système (10) de la revendication 3,
dans lequel l'un des flux de données représente une activité cardiaque,
dans lequel le sous-système processeur (40) est en outre configuré pour déterminer si le flux de données représentant l'activité cardiaque indique un rythme cardiaque rapide du patient, et pour comparer le flux de données représentant le paramètre respiratoire, le flux de données représentant les données d'activité, et le flux de données représentant l'activité cardiaque de manière synchrone, et
dans lequel le sous-système processeur (40) est en outre configuré pour, en réponse à une détermination que le flux de données représentant l'activité cardiaque indique un rythme cardiaque rapide, déterminer si le rythme cardiaque rapide coïncide ou non avec l'une d'entre une augmentation de l'activité respiratoire indiquée par le flux de données représentant le paramètre respiratoire et une augmentation d'activité physique indiquée par le flux de données représentant des données d'activité.

5. Le système (10) de la revendication 4, dans lequel le sous-système processeur (40) est en outre configuré pour :
déterminer que le rythme cardiaque rapide est un état normal en réponse à une détermination que le rythme cardiaque élevé coïncide avec l'une d'entre une augmentation de l'activité respiratoire et une augmentation de l'activité physique, et
déterminer que le rythme cardiaque élevé est un état anormal, en réponse à une détermination que le rythme cardiaque élevé ne coïncide pas avec l'une d'entre une augmentation de l'activité respiratoire et une augmentation de l'activité physique.

6. Le système (10) de la revendication 1, dans lequel le système comprend en outre un vêtement apte à recouvrir au moins une partie de la poitrine du patient.

7. Le système (10) de la revendication 6, dans lequel le premier (22a, 24a) et le second (22b, 24b) capteur se trouvent à proximité de la région de la poitrine du patient lorsque le vêtement est porté par le patient.

8. Le système (10) de la revendication 1, dans lequel le premier (22a, 24a) et le second (22b, 24b) capteur sont des bobines électromagnétiques.

9. Le système (10) de la revendication 8, dans lequel le flux de données représentant le paramètre respiratoire correspond à des signaux générés par les bobines électromagnétiques, les signaux étant représentatifs de la modification de distance entre celles-ci.

10. Un procédé d'interprétation et d'analyse d'informations relatives à un patient (100), le procédé comprenant :
la génération de flux de données représentant des paramètres d'un patient de manière continue, synchrone et simultanée ;
la réception des flux de données de manière continue, synchrone et simultanée ;
la détermination d'un paramètre contextuel à partir de la pluralité reçue de flux de données, les flux de données étant générés par des capteurs (22a, 22b ; 24a, 24b) positionnés à proximité du corps du patient, **caractérisé en ce que**
l'étape de détermination d'un paramètre contextuel comprend l'utilisation de données d'autres patients pour déterminer si les données interprétées à partir des flux de données indiquent un paramètre contextuel relatif au patient (100), tel que la présence ou l'absence d'une pathologie ou autre état médical, une réponse à un traitement, une potentialité de crise ou d'état médical imminents, un niveau de forme physique, ou une modification du niveau de forme physique.

11. Le procédé de la revendication 10, dans lequel l'un des flux de données représente un paramètre respiratoire, et dans lequel l'un des flux de données représente des données d'activité.

12. Le procédé de la revendication 11, comprenant en outre :
la comparaison du flux de données représentant le paramètre respiratoire au flux de données représentant les données d'activité de façon synchrone ; et
la détermination d'un état du patient (100) sur la base de la comparaison.

13. Le procédé de la revendication 12, comprenant en outre :
la détermination si un flux de données représentant une activité cardiaque indique un rythme cardiaque rapide du patient (100) ;
la comparaison du flux de données représentant le paramètre respiratoire, du flux de données représentant les données d'activité, et du flux de données représentant l'activité cardiaque de manière synchrone ;
la détermination, en réponse à une détermination que le flux de données représentant l'activité cardiaque indique un rythme cardiaque rapide, si le rythme cardiaque rapide coïncide ou non avec l'une d'entre une augmentation de l'activité respiratoire indiquée par le flux de données représentant le paramètre respiratoire et une augmentation d'activité physique indiquée par le flux de données représentant des données d'activité.

14. Le procédé de la revendication 13, comprenant en outre :
la détermination, en réponse à une détermination que le rythme cardiaque élevé coïncide avec l'une d'entre une augmentation de l'activité respiratoire et une augmentation de l'activité physique, que le rythme cardiaque rapide est un état normal, et
la détermination, en réponse à une détermination que le rythme cardiaque élevé ne coïncide pas avec l'une d'entre une augmentation de l'activité respiratoire et une augmentation de l'activité physique, que le rythme cardiaque élevé est un état anormal.
